# EUROPÄISCHE PATENTANMELDUNG

(11) **EP 3 536 218 A1**
(43) Veröffentlichungstag der Anmeldung: **11.09.2019**
(21) Anmeldenummer: 19153734.9
(22) Anmeldetag: 25.01.2019
(51) Int. Cl.: A61B 1/00, A61B 1/267

(54) **LARYNGOSKOP UND SPATEL FÜR EIN LARYNGOSKOP**

(30) Priorität: 09.03.2018 DE 102018105538
(71) Anmelder: Karl Storz SE & Co. KG, 78532 Tuttlingen (DE)
(72) Erfinder: Attinger, Jürg, 8260 Stein am Rhein (CH)

(57) **Zusammenfassung**

Ein Spatel (20) für ein Laryngoskop (10) umfasst ein proximalen Ende (24), das mit einem Griffbauteil (16) mechanisch verbunden oder verbindbar ist, ein distales Ende (22) und eine Öffnung (52) nahe dem distalen Ende (22) des Spatels (20). Die Öffnung (52) ist derart angeordnet und ausgebildet, dass durch die Öffnung (52) hindurch ein Blick von der bei vorgesehener Verwendung des Spatels (20) dem Gaumen eines Patienten zuzuwendenden Seite (28) zu der bei vorgesehener Verwendung des Spatels (20) dem Zungengrund des Patienten zuzuwendenden Seite (26) möglich ist.

## Beschreibung

Die vorliegende Erfindung ist auf einen Spatel für ein Laryngoskop und auf ein Laryngoskop mit einem solchen Spatel bezogen.

Bei (genauer: unmittelbar vor) einer endotrachealen Intubation wird die Epiglottis (deutsch: der Kehldeckel) in der Regel mittels eines Laryngoskops vom Eingang des Larynx (deutsch: Kehlkopf) abgehoben und in seiner den Larynx nicht verschließenden Position immobilisiert. Die dazu erforderliche präzise Positionierung des distalen Endes des Laryngoskops auf oder unter der Epiglottis wird vereinfacht, wenn das Laryngoskop mit einer bildgebenden Einrichtung, insbesondere einer Kamera nahe seinem distalen Ende ausgestattet ist. Trotzdem erfordert die Durchführung der Maßnahme nicht nur anatomische Kenntnisse, sondern auch Geschick und Erfahrung. In der Regel wird das Laryngoskop in einem iterativen Verfahren mehrfach vor- und zurückbewegt bis sein distales Ende eine geeignete Position einnimmt.

Eine Aufgabe der vorliegenden Erfindung besteht darin, einen verbesserten Spatel für ein Laryngoskop und ein verbessertes Laryngoskop zu schaffen.

Diese Aufgabe wird durch die Gegenstände der unabhängigen Ansprüche gelöst.

Weiterbildungen sind in den abhängigen Ansprüchen angegeben.

Ein Spatel für ein Laryngoskop umfasst ein proximales Ende, das mit einem Griffbauteil mechanisch verbunden oder verbindbar ist, ein distales Ende und eine Öffnung nahe dem distalen Ende des Spatels, wobei die Öffnung derart angeordnet und ausgebildet ist, dass durch die Öffnung hindurch ein Blick von der bei der vorgesehenen Verwendung des Spatels dem Gaumen eines Patienten zuzuwendenden Seite zu der bei der vorgesehenen Verwendung des Spatels dem Zungengrund des Patienten zuzuwendenden Seite möglich ist.

Der Spatel ist insbesondere für ein Intubationslaryngoskop für Anästhesie, Notfallmedizin oder Intensivmedizin oder für ein Laryngoskop für die Larynxchirurgie oder andere Aufgaben innerhalb der Hals-Nasen-Ohren-Heilkunde bzw. Oto-Rhino-Laryngologie (englisch: otorhinolaryngology) vorgesehen oder Teil eines solchen Laryngoskops. Der Spatel kann von der Öffnung abgesehen einem herkömmlichen Spatel - beispielsweise nach Macintosh, Miller, Dörges oder McCoy ähneln.

Das proximale Ende des Spatels kann mechanisch starr oder gelenkig mit dem Griffbauteil verbunden oder verbindbar sein. Das distale Ende des Spatels ist dafür vorgesehen und ausgebildet, bei der vorgesehenen Verwendung des Spatels in den Rachen des Patienten eingeführt zu werden.

Der Spatel kann zur Verwendung mit einem (insbesondere flexiblen) Endoskop oder einer anderen Vorrichtung zur Erfassung und Wiedergabe eines Bilds von der Umgebung des distalen Endes des Spatels vorgesehen und ausgebildet sein. Alternativ kann der Spatel nahe seinem distalen Ende eine Kamera oder ein Objektiv und ein distales Ende eines geordneten Bündels von Lichtleitfasern oder eines Relaislinsensystems oder einer anderen Einrichtung zum Übertragen des von dem Objektiv erfassten Bilds aufweisen. Alternativ kann der Spatel vorgesehen und ausgebildet sein, um nahe seinem distalen Ende eine Kamera mit einem Objektiv und einem Bildsensor aufzunehmen. Das distale Ende des Endoskops bzw. das Objektiv bzw. die Kamera ist dabei an oder nahe einer bei der vorgesehenen Verwendung des Spatels dem Gaumen des Patienten zuzuwendenden Seite des Spatels angeordnet und hätte ohne die Öffnung keine Sicht zu der bei der vorgesehenen Verwendung des Spatels dem Zungengrund des Patienten zuzuwendenden Seite.

Die Öffnung ermöglicht eine Sicht zu der bei der vorgesehenen Verwendung des Spatels dem Zungengrund des Patienten zuzuwendende Seite des Spatels. Dies kann eine korrekte Positionierung des Spatels vereinfachen und damit insbesondere eine schnellere und zuverlässigere endotracheale Intubation ermöglichen. Auch für andere Anwendungen kann die mögliche Sicht durch die Öffnung hindurch auf die bei der vorgesehenen Verwendung dem Zungengrund des Patienten zuzuwendende Seite vorteilhaft sein.

Bei einem Spatel, wie er hier beschrieben ist, ist die Öffnung insbesondere nicht durch ein Bauteil aus einem optisch transparenten Material verschlossen.

Ein Fensterbauteil aus einem optisch transparenten Material mag mechanisch vorteilhaft erscheinen. An den Oberflächen eines solchen Fensterbauteils auftretende Reflexionen (einschließlich Totalreflexion am Übergang von dem transparenten Material zu Luft) können jedoch die Konstruktion des Fensterbauteils erheblich erschweren und/oder eine Beobachtung durch das Fensterbauteil hindurch unmöglich machen. Das Vorsehen einer einfachen Öffnung nahe dem distalen Ende des Spatels ermöglicht hingegen eine klare und von Reflexionen unbeeinträchtigte Beobachtung durch die Öffnung hindurch.

Bei einem Spatel, wie er hier beschrieben ist, weist die Öffnung insbesondere eine rechteckige oder im Wesentlichen rechteckige Gestalt auf.

Die Öffnung ist rechteckig oder im Wesentlichen rechteckig, wenn ihr Flächeninhalt nicht geringer als vier Fünftel oder neun Zehntel oder 19 Zwanzigstel des Flächeninhalts eines umschreibenden Rechtecks ist. Wenn die Öffnung durch Ränder mit geneigten Flanken begrenzt ist, ist die Öffnung rechteckig oder im Wesentlichen rechteckig im obigen Sinne, wenn das genannte Kriterium für einen Schnitt entlang einer Ebene oder einer der Wölbung des Spatels entsprechend gewölbten Fläche gilt.

Bei einem Spatel, wie er hier beschrieben ist, weist die Öffnung insbesondere eine kreisförmige oder im Wesentlichen kreisförmige oder elliptische oder im Wesentlichen elliptische Gestalt auf.

Die Öffnung ist kreisförmig oder im Wesentlichen kreisförmig oder elliptisch oder im Wesentlichen elliptisch, wenn ihr Flächeninhalt nicht geringer als vier Fünftel oder neun Zehntel oder 19 Zwanzigstel des Flächeninhalts eines umschreibenden Kreises oder einer umschreibenden Ellipse ist.

Bei einem Spatel, wie er hier beschrieben ist, ist die Gestalt der Öffnung insbesondere an die typische Gestalt der menschlichen Epiglottis angepasst.

Wenn der Spatel für erwachsene Patienten vorgesehen und ausgebildet ist, ist die Gestalt der Öffnung insbesondere an die typische Gestalt der Epiglottis einer erwachsenen Patientin oder eines erwachsenen Patienten angepasst. Wenn der Spatel für kindliche oder jugendliche Patienten vorgesehen und ausgebildet ist, ist die Gestalt der Öffnung insbesondere an die typische Gestalt der Epiglottis eines kindlichen oder jugendlichen Patienten angepasst. Wenn der Spatel für Patienten mit einer vorbestimmten Körpergröße oder eine vorbestimmten Größe des Kopfes oder des Rachens vorgesehen und ausgebildet ist, ist die Gestalt der Öffnung insbesondere an die typische Gestalt einer Patientin oder eines Patienten mit der vorbestimmten Körpergröße oder der vorbestimmten Größe des Kopfes oder des Rachens angepasst.

Die Anpassung der Gestalt der Öffnung an die typische Gestalt der menschlichen Epiglottis kann eine formschlüssige Justage der Positionierung des Spatels ermöglichen. Dies gilt insbesondere, wenn der Spatel posterior der Epiglottis angeordnet wird, um diese nach anterior zu drücken.

Ein Spatel, wie er hier beschrieben ist, mit mehreren Öffnungen umfasst insbesondere ferner einen Steg zwischen zwei Öffnungen.

Mehrere Öffnungen des Spatels sind insbesondere durch einen oder mehrere Stege voneinander getrennt. Dieselbe Gesamtsituation kann so beschrieben werden, dass eine GesamtÖffnung durch einen oder mehrere Stege in mehrere Teilöffnungen unterteilt ist. Die Sprosse oder die Sprossen sind insbesondere wie die Sprosse oder die Sprossen eines Sprossenfensters angeordnet, wobei im Gegensatz zu einem Sprossenfenster kein Glas in den Öffnungen vorgesehen ist.

Ein oder mehrere Stege können ein Eindringen oder ein Hineinwölben von Gewebeoberflächen in die Öffnung verhindern oder reduzieren oder den Blick zu der bei der vorgesehenen Verwendung des Spatels dem Zungengrund zugewandten Seite des Spatels hin wesentlich einzuschränken. Ein oder mehrere Stege können ferner die mechanische Stabilität des Spatels erhöhen.

Bei einem Spatel, wie er hier beschrieben ist, ist der Steg insbesondere parallel oder im Wesentlichen parallel zu der Längsrichtung des Spatels oder orthogonal oder im Wesentlichen orthogonal zu der Längsrichtung des Spatels oder in einem Winkel, der nicht kleiner als 30 Grad und nicht größer als 60 Grad ist, zu der Längsrichtung des Spatels angeordnet.

Ein Steg ist parallel oder im Wesentlichen parallel zu der Längsrichtung des Spatels angeordnet, wenn der Steg mit der Längsrichtung einen Winkel einschließt, der nicht größer als 10 Grad oder nicht größer als 20 Grad oder nicht größer als 30 Grad ist. Der Steg ist orthogonal oder im Wesentlichen orthogonal zu der Längsrichtung des Spatels angeordnet, wenn der Winkel zwischen dem Steg und der Längsrichtung des Spatels nicht kleiner als 60 Grad oder nicht kleiner als 70 Grad oder nicht kleiner als 80 Grad ist.

Ein Spatel, wie er hier beschrieben ist, umfasst insbesondere ferner einen weiteren Steg, der parallel zu dem Steg angeordnet ist oder den Steg kreuzt.

Beispielsweise können zwei einander kreuzende Stege, von denen einer im Wesentlichen parallel und der andere im Wesentlichen orthogonal zu der Längsrichtung des Spatels angeordnet ist, eine im Wesentlichen rechteckige Gesamtöffnung in vier jeweils im Wesentlichen rechteckige Teilöffnungen unterteilen. Alternativ können beispielsweise zwei im Wesentlichen parallele Stege eine im Wesentlichen rechteckige Gesamtöffnung in drei Teilöffnungen unterteilen.

Ein Spatel, wie er hier beschrieben ist, umfasst insbesondere ferner ein Netz oder Gitter aus mehreren Stegen, die einander kreuzen oder deren Enden miteinander verbunden sind.

Jeweils die Enden von drei oder vier oder mehr Stegen bilden einen Knoten. Jeweils drei, vier oder mehr Stege bilden eine Masche des Netzes oder Gitters.

Das Netz oder Gitter ist insbesondere starr, d. h. unelastisch ausgebildet. Dazu sind die Stege insbesondere aus einem starren oder unelastischen Material gebildet und ihre Enden starr miteinander verbunden. Alternativ kann das Netz oder Gitter elastisch ausgebildet sind. Dazu weisen die Stege insbesondere ein elastisches Material auf und/oder ihre Enden sind elastisch miteinander verbunden.

Bei einem Spatel, wie er hier beschrieben ist, weisen insbesondere eine oder mehrere oder alle Öffnungen jeweils im Wesentlichen eine quadratische oder rechteckige oder rhombische oder hexagonale oder andere polygonale Gestalt auf.

Bei einem Spatel, wie er hier beschrieben ist, bilden die Stege insbesondere eine wabenförmige Struktur.

Dazu umschließen jeweils sechs gerade oder im Wesentlichen gerade Stege eine Öffnung oder Teilöffnung und miteinander verbundene Enden von jeweils drei Stegen bilden einen Knoten.

Bei einem Spatel, wie er hier beschrieben ist, ist insbesondere ein Bereich des Spatels oder der gesamte Spatel durch Stege gebildet, zwischen denen die Öffnung oder mehrere Öffnungen angeordnet sind.

Bei einer netz- oder gitterförmigen Anordnung der Stege sind die Öffnungen durch die Maschen des Netzes oder Gitters gebildet. Wenn nicht der gesamte Spatel, sondern nur ein Bereich des Spatels durch Stege gebildet ist, ist dieser Bereich insbesondere ein distaler Bereich des Spatels.

Bei einem Spatel, wie er hier beschrieben ist, ist der durch Stege gebildete Bereich insbesondere zumindest entweder streifenförmig oder rechteckig.

Die geraden oder im Wesentlichen geraden Randabschnitte des streifenförmigen und/oder rechteckigen durch Stege gebildeten Bereichs sind insbesondere parallel oder orthogonal zur Längsrichtung des Spatels.

Bei einem Spatel, wie er hier beschrieben ist, ist die Öffnung zumindest entweder distal einer Lichtaustrittsfläche, durch die bei der vorgesehenen Verwendung des Spatels Beleuchtungslicht austritt, oder distal einer Lichteintrittsfläche, durch die hindurch bei der vorgesehenen Verwendung des Spatels ein Bild der Umgebung des distalen Endes des Spatels erfasst werden kann, angeordnet.

Die Lichtaustrittsfläche ist insbesondere eine Lichtaustrittsfläche an einem mit dem Spatel dauerhaft und starr verbundenen Fensterbauteil oder Lichtleiter oder Lichtleiterbündel. Alternativ kann die Lichtaustrittsfläche eine Lichtaustrittsfläche an einem mit dem Spatel nicht dauerhaft verbundenen Fensterbauteil oder Lichtleiter oder Lichtleiterbündel in einer vorbestimmten Position sein. In diesem Fall ist die vorbestimmte Position der Lichtaustrittsfläche insbesondere formschlüssig definiert, beispielsweise durch einen mechanischen Anschlag, bis zu dem eine Beleuchtungsvorrichtung mit der Lichtaustrittsfläche relativ zu dem Spatel nach distal bewegt werden kann, und der eine weitere Bewegung nach distal verhindert.

Die Lichteintrittsfläche ist insbesondere eine Lichteintrittsfläche an einem Objektiv oder an einem Fensterbauteil, hinter dem eine Kamera oder ein Objektiv und ein Bildsensor oder ein Objektiv und ein distales Ende eines geordneten Bündels von Lichtleitern oder eines Relaislinsensystems oder einer anderen Bildübertragungseinrichtung angeordnet ist. Das Objektiv oder das Fensterbauteil mit der Lichteintrittsfläche kann dauerhaft mit dem Spatel verbunden sein, beispielsweise als Teil einer in den Spatel integrierten Kamera oder eines in den Spatel integrierten Endoskops oder einer anderen in den Spatel integrierten Bilderfassungseinrichtung oder Bildübertragungseinrichtung. Alternativ kann die Lichteintrittfläche vorgesehen sein an einer Kamera oder einem Endoskop oder einer anderen Bilderfassungseinrichtung oder Bildübertragungseinrichtung, die nicht dauerhaft mit dem Spatel mechanisch verbundenen, sondern mit dem Spatel kombinierbarn und mechanisch verbindbar ist. Die vorbestimmte Position der Lichteintrittsfläche ist insbesondere formschlüssig definiert, beispielsweise durch einen mechanischen Anschlag, bis zu dem die Kamera oder das Endoskop oder die Bildübertragungseinrichtung oder die Bilderfassungseinrichtung mit der Lichteintrittsfläche relativ zu dem Spatel nach distal bewegt werden kann, und der eine weitere Bewegung nach distal verhindert.

Ein Laryngoskop umfasst einen Spatel, wie er hier beschrieben ist, und ein Griffbauteil, das mit dem proximalen Ende des Spatels verbunden oder verbindbar ist.

### Kurzbeschreibung der Figuren

Nachfolgend werden Ausführungsformen anhand der beigefügten Figuren näher erläutert. Es zeigen:
- Figur 1: eine schematische Darstellung eines Laryngoskops;
- Figur 2: eine schematische Darstellung eines Schnitts durch einen Spatel des Laryngoskops aus Figur 1;
- Figur 3: eine schematische Darstellung eines weiteren Laryngoskops;
- Figur 4: eine schematische Darstellung eines Schnitts durch einen Spatel des Laryngoskops aus Figur 3;
- Figur 5: eine schematische Darstellung eines Schnitts durch einen weiteren Spatel;
- Figur 6: eine schematische Darstellung eines Schnitts durch einen weiteren Spatel;
- Figur 7: eine schematische Darstellung eines Schnitts durch einen weiteren Spatel;
- Figur 8: eine schematische Darstellung eines Schnitts durch einen weiteren Spatel;
- Figur 9: eine schematische Darstellung eines Schnitts durch einen weiteren Spatel;
- Figur 10: eine schematische Darstellung eines Schnitts durch einen weiteren Spatel.

### Beschreibung der Ausführungsformen

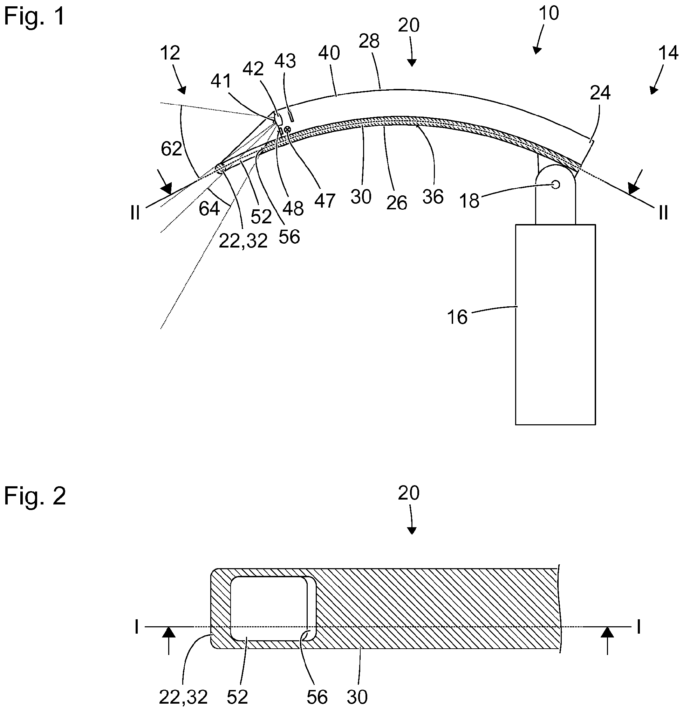

Figur 1 zeigt eine schematische Darstellung eines Laryngoskops 10, das beispielsweise zur Unterstützung der endotrachealen Intubation geeignet ist. Das Laryngoskop 10 weist ein distales Ende 12 auf, das dafür vorgesehen und ausgebildet ist, in den Rachen eines Patienten eingeführt zu werden. Ferner weist das Endoskop ein proximales Ende 14 auf, das im Wesentlichen durch einen Griff 16 gebildet ist. Der Griff 16 ist - bei dem dargestellten Beispiel durch ein arretierbares Gelenk 18 - mit einem gekrümmten Spatel 20 verbunden. Ein distales Ende 22 des Spatels 20 bildet das distale Ende 12 des Laryngoskops 10. Ein proximales Ende 24 des Spatels 20 ist durch das erwähnte Gelenk 18 mit dem Griff 16 verbunden. Alternativ kann das proximale Ende 24 des Spatels 20 mechanisch starr - und zwar permanent oder lösbar - mit dem Griff 16 verbunden sein.

Der Spatel 20 weist eine, bei der vorgesehenen Verwendung des Laryngoskops 10 der Zunge eines Patienten zuzuwendende Seite 26 und eine davon abgewandte, bei der vorgesehenen Verwendung dem Gaumen eines Patienten zuzuwendende Seite 28 auf. Die der Zunge eines Patienten zuzuwendende Seite 26 des Spatels 20 wird durch eine balkenförmige Struktur 30 gebildet. Die balkenförmige Struktur weist an ihrem distalen Ende eine Verdickung 32 auf, die das distale Ende 22 des Spatels 20 bildet. Die Verdickung 32 ermöglicht oder unterstützt mit möglichst großen Krümmungsradien eine atraumatische Verwendung des Laryngoskops 10.

Querschnitte der balkenförmigen Struktur 30 in Ebenen orthogonal zu der Zeichenebene der Figur 1 sind - von der Verdickung 32 an ihrem distalen Ende und nachfolgend beschriebenen Merkmalen abgesehen - im Wesentlichen rechteckig mit abgerundeten Ecken. Die balkenförmige Struktur 30 ist entsprechend der Krümmung des gesamten Spatels 20 gekrümmt. An der der Zunge eines Patienten zuzuwendenden Seite 26 des Spatels 20 weist die balkenförmige Struktur 30 einen zur Anlage an der Zunge des Patienten vorgesehenen Oberflächenbereich 36 auf.

Der Spatel 20 umfasst ferner einen Hohlkörper 40, der sich von dem proximalen Ende 24 über einen großen Teil der Länge des Spatels 20 bis zu einem Ort an einem vorbestimmten Abstand zu dem distalen Ende 22 des Spatels 20 erstreckt. Während die balkenförmige Struktur 30 die der Zunge eines Patienten zuzuwendende Seite 26 des Spatels 20 bildet, bildet der Hohlkörper 40 die dem Gaumen des Patienten zuzuwendende Seite 28 des Spatels 20. Der Hohlkörper 40 nimmt nicht die gesamte Breite des Spatels 20 ein, sondern von proximal betrachtet beispielsweise nur die rechte Seite, so dass die balkenförmige Struktur 30 an der linken, bei der Darstellung in Figur 1 dem Betrachter zugewandten Seite übersteht.

Bei der Darstellung in Figur 1 ist die balkenförmige Struktur 30 in einem Schnitt entlang einer Ebene, die außerhalb des Hohlkörpers 40 liegt, gezeigt. Der Hohlkörper 40 ist also in Draufsicht dargestellt. Abweichend davon sind jedoch eine Lichteintrittsfläche 41 des Spatels 20, ein Objektiv 42 und ein Bildsensor 43 proximal des Objektivs 42 an dem distalen Ende des Hohlkörpers 40 angedeutet, obwohl zumindest das Objektiv 42 weitgehend und der Bildsensor 43 vollständig innerhalb des Hohlkörpers 40 und damit aus der in Figur 1 dargestellten Blickrichtung nicht sichtbar sind. Die Lichteintrittsfläche 41 des Spatels 20 ist bei dem angedeuteten Beispiel durch eine Lichteintrittsfläche des Objektivs 42 gebildet.

Das Objektiv 42 und der Bildsensor 43 bilden eine in das Laryngoskop 10 integrierte Kamera und stellen ein Beispiel einer Bilderfassungseinrichtung zum Erfassen eines Bilds der Umgebung des distalen Endes 22 des Spatels 20 dar. Diese Bilderfassungseinrichtung kann anstelle des Bildsensors 43 ein distales Ende eines geordneten Bündels von Lichtleitfasern, eines Relaislinsensystems oder einer anderen Einrichtung zum Übertragen des von dem Objektiv 42 erzeugten Bilds zu dem proximalen Ende 14 des Laryngoskops 10 aufweisen.

Die Kamera oder die andere Bilderfassungseinrichtung zum Erfassen eines Bilds der Umgebung des distalen Endes 22 des Spatels 20 kann mit dem Spatel 20 dauerhaft und nicht ohne Weiteres lösbar mechanisch verbunden sein. Alternativ kann die Kamera oder die andere Bilderfassungseinrichtung lösbar mit dem Spatel 20 verbunden sein, beispielsweise indem sie von proximal bis zu einem Anschlag in den Hohlkörper 40 eingeschoben wird. Die Lichteintrittsfläche 41 kann in diesem Fall eine Lichteintrittsfläche eines Fensterbauteils, das den Hohlkörper 40 distal fluiddicht oder hermetisch verschließt, sein. Alternativ ist die Lichteintrittsfläche 41 die Lichteintrittsfläche der Kamera oder der anderen Bilderfassungseinrichtung. In letzterem Fall ist die Position der Lichteintrittsfläche 41 relativ zu dem Spatel 20 und dessen distalem Ende 22 insbesondere durch die vorgesehene Bauart und die beispielsweise formschlüssig definierte Position und Orientierung der Kamera oder der anderen Bilderfassungseinrichtung relativ zu dem Spatel 20 definiert.

Obwohl, wie erwähnt, in Figur 1 der Hohlkörper 40 nicht in einem Schnitt dargestellt ist, sind ferner eine Lichtquelle 47 und eine Lichtaustrittsfläche 48 an dem distalen Ende des Hohlkörpers 40 angedeutet. Die Lichtquelle 47 umfasst beispielsweise eine oder mehrere Leuchtdioden. Von der Lichtquelle 47 erzeugtes Beleuchtungslicht tritt durch die Lichtaustrittsfläche 48 aus dem Spatel aus und beleuchtet die Umgebung des distalen Endes 22 des Spatels 20. Um eine Abschattung durch das distale Ende 22 des Spatels 20 zu verhindern oder zu vermindern, kann die Lichtaustrittsfläche 48 die Lichteintrittsfläche 41 teilweise oder vollständig umgeben oder es können mehrere Lichtaustrittsflächen vorgesehen sein, die insbesondere an gegenüberliegenden Seiten der Lichteintrittsfläche 41 angeordnet sein können.

Der Spatel 20 weist nahe seinem distalen Ende 22 eine Öffnung 52 in der balkenförmigen Struktur 30 auf. Die Öffnung 52 ermöglicht, dass mittels des Objektivs 42 und des Bildsensors 43 nicht nur ein Sichtfeld 62, sondern auch ein weiteres Sichtfeld 64 an der der Zunge eines Patienten zuzuwendenden Seite 26 des Spatels 20 betrachtet oder beobachtet werden kann. Dies kann die korrekte Positionierung des distalen Endes 22 des Spatels 20 deutlich vereinfachen.

Der proximale Randabschnitt der Öffnung 52 in der balkenförmigen Struktur 30 weist eine geneigte Flanke 56 auf. Diese Neigung ermöglicht ein besonders günstiges Verhältnis zwischen der Größe des zur Anlage an der Zunge eines Patienten vorgesehenen Oberflächenbereichs 36 der balkenförmigen Struktur 30 und der Größe des zweiten Sichtfelds 64.

Die Öffnung 52 ist insbesondere nicht durch ein Fensterbauteil aus einem transparenten Bauteil verschlossen, so dass weder Reflexionen noch feste oder flüssige Ablagerungen an den Oberflächen eines solchen Fensterbauteils die Sicht durch die Öffnung 52 hindurch beeinträchtigen können.

Figur 2 zeigt eine vergrößerte schematische Darstellung eines Schnitts entlang der in Figur 1 angedeuteten gekrümmten und der Form der balkenförmigen Struktur 30 folgenden Fläche II-II. Die Position der Schnittebene I-I, in der die balkenförmige Struktur 30 in Figur 1 dargestellt ist, ist in Figur 2 angedeutet.

In Figur 2 ist die länglich-rechteckige Grundgestalt der balkenförmigen Struktur erkennbar. Der Querschnitt der balkenförmigen Struktur 30 in einer Ebene orthogonal zu der Zeichenebene der Figur 1 und der Schnittfläche II-II der Figur 2 ist - wie bereits erwähnt - insbesondere flach rechteckig mit abgerundeten Ecken. Auch die in Figur 2 erkennbaren Ecken an dem distalen Ende 32 der balkenförmigen Struktur 30 und damit an dem distalen Ende 22 des Spatels 20 sind abgerundet.

Die Öffnung 52 in der balkenförmigen Struktur 30 des Spatels 20 ist bei dem dargestellten Beispiel ebenfalls im Wesentlichen rechteckig mit abgerundeten Ecken. Die geneigte Flanke 56 des proximalen geraden Randabschnitts ist, wie erwähnt, gegenüber der in Figur 2 dargestellten Schnittfläche II-II geneigt.

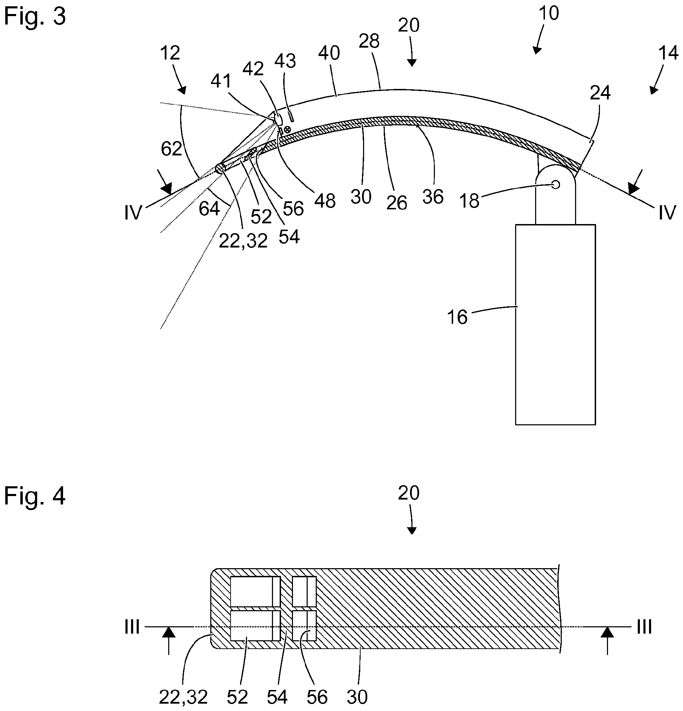

Figur 3 zeigt eine schematische Darstellung eines weiteren Laryngoskops 10, das in einigen Merkmalen, Eigenschaften und Funktionen dem anhand der Figuren 1 und 2 dargestellten Laryngoskop ähnelt. Die Art der Darstellung in Figur 3 entspricht der Art der Darstellung in Figur 1. Nachfolgend sind insbesondere Merkmale, Eigenschaften und Funktionen des in Figur 3 gezeigten Laryngoskops 10 gezeigt, in denen dieses sich von dem anhand der Figuren 1 und 2 dargestellten Laryngoskop unterscheidet.

Das in Figur 3 gezeigte Laryngoskop 10 unterscheidet sich von dem anhand der Figuren 1 und 2 dargestellten Laryngoskop insbesondere dadurch, dass die Öffnung 52 nahe dem distalen Ende 22 des Spatels 20 durch einen Steg 54 in mehrere Teilöffnungen unterteilt ist.

Figur 4 ist eine schematische Darstellung eines Schnitts durch die balkenförmige Struktur 30 des Spatels 20 des anhand der Figur 3 dargestellten Laryngoskops 10 entlang der in Figur 3 angedeuteten gekrümmten und der Gestalt der balkenförmigen Struktur 30 folgenden Schnittfläche IV-IV. Die Position der Schnittebene III-III, in der die balkenförmige Struktur 30 in Figur 3 dargestellt ist, ist in Figur 4 angedeutet.

In Figur 4 ist erkennbar, dass zwei einander kreuzende und somit durchdringende Stege 54 die Öffnung 52 in dem Spatel 20 in vier Teilöffnungen unterteilen. Die dadurch vorliegende Struktur kann alternativ so beschrieben werden, dass der Spatel 20, nämlich dessen balkenförmige Struktur 30 mehrere Öffnungen 52 aufweist, die durch Stege 54 voneinander getrennt sind.

Die in Figur 4 gezeigte Struktur kann so beschrieben werden, dass ein in Längsrichtung (in Figur 4: horizontal) angeordneter Steg 54 und ein in Querrichtung (in Figur 4: vertikal) angeordneter Steg einander kreuzen oder durchdringen. Alternativ kann die in Figur 4 dargestellte Struktur so beschrieben werden, dass je ein Ende zweier in Längsrichtung des Spatels 20 angeordneter Stege und je ein Ende zweier in Querrichtung angeordneter Stege einen Knoten bilden. Auch die äußeren Ränder der balkenförmigen Struktur 30 seitlich (in Figur 4: unter oder über) den Öffnungen 52 und der distale (in Figur 4: linke) Rand der balkenförmigen Struktur 30 können als Stege beschrieben werden. In dieser Beschreibung besteht der gesamte distale (in Figur 4: linke) Endbereich der balkenförmigen Struktur 30 aus einem Netz oder Gitter aus mehreren Stegen 54, die einander kreuzen oder deren Enden miteinander mechanisch starr verbunden sind.

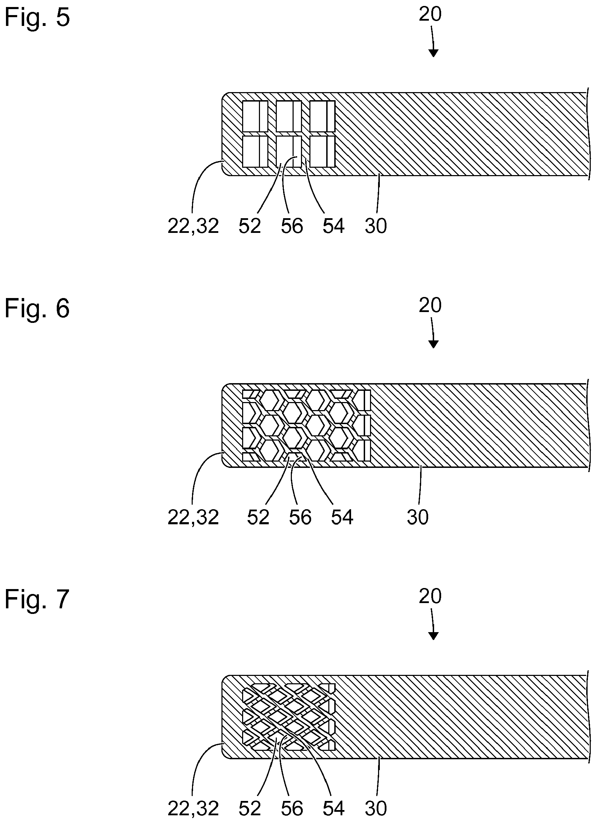

Figur 5 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 4 dargestellten Laryngoskope ähneln oder gleichen können. Die Art der Darstellung in Figur 5 entspricht der Art der Darstellung in den Figuren 2 und 4. Dargestellt ist also ein Schnitt durch eine gekrümmte balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 4 dargestellten Spateln unterscheidet.

Bei dem in Figur 5 dargestellten Spatel 20 weist die balkenförmige Struktur 30 eine größere Anzahl von Öffnungen oder Teilöffnungen 52, die durch ein Netz oder Gitter von Stegen 54 voneinander getrennt sind, auf. Zwei Querstege und ein Längssteg, die einander kreuzen oder durchdringen trennen insgesamt sechs Öffnungen oder Teilöffnungen 52 voneinander, von denen jeweils zwei nebeneinander und jeweils drei hintereinander angeordnet sind.

Figur 6 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 5 dargestellten Laryngoskope ähneln oder gleichen können. Die Art der Darstellung in Figur 6 entspricht der Art der Darstellung in den Figuren 2, 4 und 5. Dargestellt ist also ein Schnitt durch eine balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 5 dargestellten Spateln unterscheidet.

Bei dem in Figur 6 dargestellten Beispiel bilden die Stege 54 ein hexagonales Gitter, ähnlich einer von Bienen erzeugten Wabenstruktur. Die durch die Stege 54 voneinander getrennten Öffnungen oder Teilöffnungen 52, die nicht am Rand liegen, sind jeweils sechseckig.

Figur 7 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 6 dargestellten Laryngoskope ähneln oder gleichen können. Die Art der Darstellung in Figur 7 entspricht der Art der Darstellung in den Figuren 2 und 4 bis 6. Dargestellt ist also ein Schnitt durch eine balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 4 dargestellten Spateln unterscheidet.

Bei dem in Figur 7 gezeigten Beispiel bilden die Stege 54 ein rhombisches Gitter. Die durch die Stege 54 voneinander getrennten Öffnungen oder Teilöffnungen 52, die nicht am Rand liegen, weisen jeweils eine im Wesentlichen rhombische oder rautenförmige Gestalt auf.

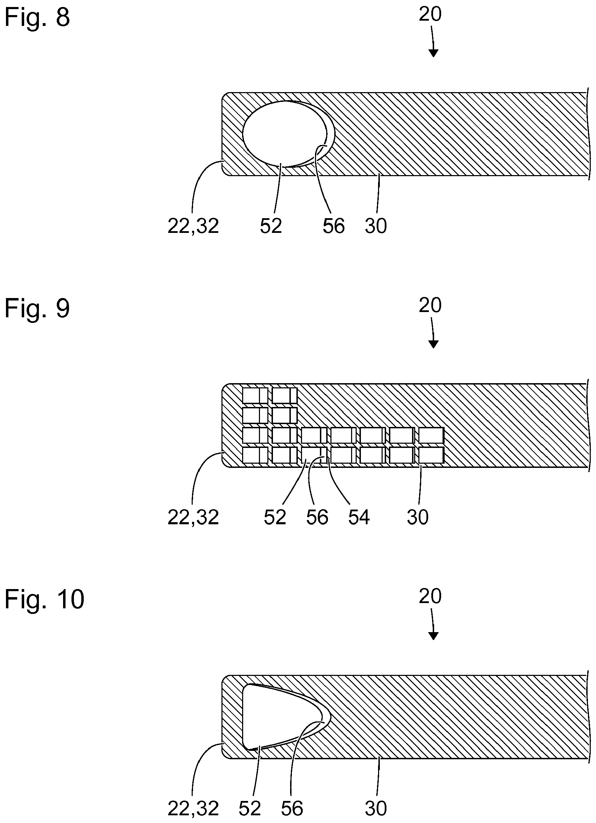

Figur 8 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 7 dargestellten Laryngoskope ähneln oder gleichen können. Die Art der Darstellung in Figur 8 entspricht der Art der Darstellung in den Figuren 2 und 4 bis 7. Dargestellt ist also ein Schnitt durch eine balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 7 dargestellten Spateln unterscheidet.

Bei dem in Figur 8 gezeigten Beispiel weist der Spatel 20 eine einzige elliptische Öffnung 52 in der balkenförmigen Struktur 30 auf.

Figur 9 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 8 dargestellten Laryngoskope ähneln oder gleichen können. Die Art der Darstellung in Figur 9 entspricht der Art der Darstellung in den Figuren 2 und 4 bis 8. Dargestellt ist also ein Schnitt durch eine balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 8 dargestellten Spateln unterscheidet.

Bei dem in Figur 9 gezeigten Beispiel weist der Spatel 20 ähnlich wie bei den anhand der Figuren 4 und 5 dargestellten Beispielen mehrere jeweils im Wesentlichen rechteckige Öffnungen oder Teilöffnungen 52 auf, die zusammengenommen einen L-förmigen Bereich des Spatels 20 einnehmen. Ein Schenkel des L-förmigen Bereichs, der von den Öffnungen oder Teilöffnungen 52 eingenommen wird, ist distal des distalen Endes des in den Figuren 2 und 4 bis 9 nicht dargestellten Hohlkörpers 40 und damit distal der Lichteintrittsfläche 41 und der Lichtaustrittsfläche 48 (vgl. Figuren 1 und 2) angeordnet. Dieser Schenkel des L-förmigen Bereichs erstreckt sich über die gesamte Breite oder im Wesentlichen über die gesamte Breite des Spatels 20. Der andere Schenkel des L-förmigen Bereichs, in dem die Öffnungen oder Teilöffnungen 52 angeordnet sind, erstreckt sich zumindest teilweise neben dem in den Figuren 2 und 4 bis 9 nicht dargestellten Hohlkörper 40 (vgl. Figuren 1 und 3) des Spatels 20 und kann beispielsweise einem neben dem Hohlkörper in den Rachen eines Patienten eingeführten Endoskop eine Sicht zu der Zunge oder dem Zungengrund des Patienten ermöglichen.

Bei dem in Figur 9 dargestellten Beispiel sind die Flanken 56 der Ränder der Öffnungen oder Teilöffnungen 52 unterschiedlich geneigt. Die Flanken proximaler Öffnungen sind steil, nach distal sind die Flanken immer stärker geneigt. Dies kann die Sicht durch die Öffnungen oder Teilöffnungen 52 verbessern und/oder die mechanische Stabilität der Stege 54 vergrößern. Derart unterschiedlich geneigte Flanken 56 können auch bei den anhand der Figuren 4 bis 7 dargestellten Spateln vorgesehen sein.

Figur 10 zeigt eine schematische Darstellung einer weiteren alternativen Ausgestaltung eines Spatels 20 eines Laryngoskops mit Merkmalen, Eigenschaften und Funktionen, die den Merkmalen, Eigenschaften und Funktionen der anhand der Figuren 1 bis 9 dargestellten Laryngoskope ähneln oder gleichen können. Die Art der Darstellung in Figur 10 entspricht der Art der Darstellung in den Figuren 2 und 4 bis 9. Dargestellt ist also ein Schnitt durch eine balkenförmige Struktur 30 des Spatels 20 entlang einer Fläche, die der gekrümmten balkenförmigen Struktur 30 folgt. Nachfolgend sind insbesondere Merkmale, Eigenschaften und Funktionen des Spatels 20 dargestellt, in denen dieser sich von den anhand der Figuren 1 bis 9 dargestellten Spateln unterscheidet.

Bei dem in Figur 10 dargestellten Beispiel weist der Spatel 20 ähnlich wie bei den anhand der Figuren 2 und 8 dargestellten Beispielen nur eine einzige große Öffnung 52 auf. Im Gegensatz zu den anhand der Figuren 2 und 8 dargestellten Spateln ist bei dem in Figur 10 gezeigten Spatel 20 die Gestalt der Öffnung 52 näherungsweise an die Gestalt der menschlichen Epiglottis angepasst. Dies kann ein teilweises Eingreifen der Epiglottis in die Öffnung 52 und damit einen Formschluss zwischen dem Spatel 20 und der Epiglottis ermöglichen und damit wiederum die Positionierung des Spatels 20 vereinfachen.

### Bezugszeichen

- 10: Laryngoskop
- 12: distales Ende des Laryngoskops 10
- 14: proximales Ende des Laryngoskops 10
- 16: Griff des Laryngoskops 10
- 18: Gelenk zwischen Griff 16 und Spatel 20
- 20: Spatel des Laryngoskops 10
- 22: distales Ende des Spatels 20
- 24: proximales Ende des Spatels 20
- 26: der Zunge eines Patienten zuzuwendende Seite des Spatels 20
- 28: dem Gaumen eines Patienten zuzuwendende Seite des Spatels 20
- 30: balkenförmige Struktur des Spatels 20
- 32: Verdickung am distalen Ende 22 des Spatels 20
- 36: zur Anlage an der Zunge eines Patienten vorgesehener Oberflächenbereich der balkenförmigen Struktur 30
- 40: Hohlkörper an der dem Gaumen eines Patienten zuzuwendenden Seite 28 des Spatels 20
- 41: Lichteintrittsfläche an dem Spatels 20
- 42: Objektiv an dem distalen Ende des Hohlkörpers 40, die Lichteintrittsfläche 41 bildend
- 43: Bildsensor proximal des Objektivs 42
- 47: Lichtquelle
- 48: Lichtaustrittsfläche an dem Spatel 20
- 52: Öffnung in dem Spatel 20 nahe dessen distalem Ende 22
- 54: Steg zwischen Öffnungen 52 oder die Öffnung 52 unterteilend
- 56: geneigte Flanke der Öffnung 52
- 62: erstes Sichtfeld von Objektiv 42 und Bildsensor 43
- 64: zweites Sichtfeld von Objektiv 42 und Bildsensor 43, durch Öffnung 52

## Patentansprüche

1. **Spatel** (20) für ein Laryngoskop (10), mit:
einem **proximalen Ende** (24), das mit einem Griffbauteil (16) mechanisch verbunden oder verbindbar ist;
einem **distalen Ende** (22);
einer **Öffnung** (52) nahe dem distalen Ende (22) des Spatels (20),
wobei die Öffnung (52) derart angeordnet und ausgebildet ist, dass durch die Öffnung (52) hindurch ein Blick von der bei der vorgesehenen Verwendung des Spatels (20) dem Gaumen eines Patienten zuzuwendenden Seite (28) zu der bei vorgesehener Verwendung des Spatels (20) dem Zungengrund des Patienten zuzuwendenden Seite (26) möglich ist.

2. Spatel (20) nach einem der vorangehenden Ansprüche, bei dem die Öffnung (52) **nicht** durch ein Bauteil aus einem optisch **transparenten** Material verschlossen ist.

3. Spatel (20) nach Anspruch 1, bei dem die Gestalt der Öffnung (52) **an** die typische Gestalt der menschlichen **Epiglottis angepasst** ist.

4. Spatel (20) nach einem der vorangehenden Ansprüche, mit mehreren Öffnungen (52), ferner mit:
einem **Steg** (54) zwischen zwei Öffnungen (52).

5. Spatel (20) nach einem der vorangehenden Ansprüche, ferner mit:
einem **weiteren Steg** (54), der parallel zu dem Steg (54) angeordnet ist oder den Steg (54) kreuzt.

6. Spatel (20) nach einem der vorangehenden Ansprüche, mit:
einem **Netz oder Gitter** aus mehreren Stegen (54), die einander kreuzen oder deren Enden miteinander verbunden sind.

7. Spatel (20) nach dem vorangehenden Anspruch, bei dem die Stege (54) eine **wabenförmige** Struktur bilden.

8. Spatel (20) nach einem der vorangehenden Ansprüche, bei dem ein Bereich des Spatels (20) oder der **gesamte Spatel** (20) **durch Stege** (54) **gebildet** wird, zwischen denen die Öffnung (52) oder mehrere Öffnungen (52) angeordnet sind.

9. Spatel (20) nach einem der vorangehenden Ansprüche, bei dem die Öffnung (52) zumindest entweder **distal einer Lichtaustrittsfläche** (46), durch die bei der vorgesehenen Verwendung des Spatels (20) Beleuchtungslicht austritt, oder **distal einer Lichteintrittsfläche** (41), durch die hindurch bei der vorgesehenen Verwendung des Spatels (20) ein Bild der Umgebung des distalen Endes (22) des Spatels (20) erfasst werden kann, angeordnet ist.

10. **Laryngoskop** (10) mit:
einem **Spatel** (20) nach einem der vorangehenden Ansprüche;
einem Griffbauteil (16), das mit dem proximalen Ende (24) des Spatels (20) verbunden oder verbindbar ist.
